# EUROPÄISCHE PATENTSCHRIFT

(11) **EP 2 714 249 B1**
(45) Veröffentlichungstag und Bekanntmachung des Hinweises auf die Patenterteilung: **10.01.2018**
(21) Anmeldenummer: 11725622.2
(22) Anmeldetag: 31.05.2011
(51) Int. Cl.: A61M 1/26, B01D 61/28, B01D 63/02, A61M 1/10, A61M 1/16

(54) **VORRICHTUNG FÜR DEN STOFF- UND/ODER ENERGIEAUSTAUSCH ZWISCHEN ZWEI MEDIEN**
APPARATUS FOR MASS- AND/OR ENERGY-TRANSFER BETWEEN TWO MEDIA
DISPOSITIF D'ÉCHANGE DE MATIÈRE ET/OU D'ÉNERGIE ENTRE DEUX FLUIDES

(43) Veröffentlichungstag der Anmeldung: 09.04.2014
(73) Patentinhaber: Kashefi, Ali, 52074 Aachen (DE)
(72) Erfinder: Kashefi, Ali, 52074 Aachen (DE)
(74) Vertreter: Cohausz Hannig Borkowski Wißgott
(86) Internationale Anmeldenummer: PCT/EP2011/002681
(87) Internationale Veröffentlichungsnummer: WO 2012/163372

(56) Entgegenhaltungen:
- WO-A1-2009/110652
- DE-A1-102007 010 112
- DE-A1-102010 005 135
- US-A1- 2002 143 397

## Beschreibung

Die Erfindung betrifft eine Vorrichtung für den Stoff- und/oder Energieaustausch zwischen zwei Medien, insbesondere zwischen Blut und einem Gas/Gasgemisch, (bzw. Wasser, wenn ein pumpendes Modul der Wärmetauscher der Vorrichtung ist) mit einer von dem ersten Medium, insbesondere von Blut durchströmten Kammer, in der quer, insbesondere senkrecht zur Durchströmungsrichtung des ersten Mediums ein Bündel von stoff- und/oder energiepermeablen Hohlfasern angeordnet ist, die von dem zweiten Medium durchströmbar und vom ersten Medium umströmbar sind.

Derartige Vorrichtungen können eingesetzt werden, um allgemein zwischen einem ersten und einem zweiten Medium einen Stoff- und/oder Energieaustausch zu bewirken. Dabei sind die Hohlfasern in der genannten Kammer derart angeordnet und gegenüber der Kammer gedichtet, dass das erste Medium durch die Kammer fließen kann, ohne direkt mit dem zweiten Medium in Kontakt treten zu können, welches durch die Hohlfasern strömt. Der Stoff- und/oder Energieaustausch zwischen den beiden Medien findet demnach durch die permeablen Hohlfasern statt.

Dabei ist es im Stand der Technik bekannt, dass z.B. das Hohlfaserbündel mit seiner Längserstreckung in einem Hohlprofil einliegen kann, welches die Kammer ausbildet, wobei an den axialen Enden der Kammer Anschlüsse vorgesehen sind, die jeweils mit der Vielzahl der axialen Öffnungen der Hohlfasern am jeweiligen axialen Bündelende in Fluidverbindung stehen.

In einer bevorzugten Anwendung können solche Vorrichtungen beispielsweise in der Medizintechnik eingesetzt werden, beispielsweise bei der Blutreinigung, wie z.B. der Dialyse, der Bluttrennung oder auch als künstliche Lungen, somit als sogenannte Oxygenatoren.

Bei dieser Anwendung kann das Blut als das erste Medium betrachtet werden sowie Sauerstoff als das zweite Medium, welches durch die einzelnen Hohlfasern des vorgenannten Bündels geleitet wird. Mit CO₂ angereichertes Blut kann sodann durch die Kammer geleitet werden, wodurch sich durch die unterschiedlichen Partialdrücke von Sauerstoff und CO₂ auf den beiden Seiten der stoff- und/oder energiepermeablen Hohlfasern des Bündels ein Stoffaustausch derart ergibt, dass CO₂ aus dem Blut entfernt und das Blut mit Sauerstoff aus den Hohlfasern angereichert wird. Die Hohlfasern sind bei dieser Anwendung somit CO₂- und O₂-permeabel. Eine solche Vorrichtung kann als künstliche Lunge arbeiten und demnach die Lungenfunktion eines Patienten teilweise oder auch vollständig übernehmen. In anderen, insbesondere anderen medizinischen Anwendungen können die Hohlfasern auch für andere Stoffe permeabel ausgebildet sein.

Um das erste Medium durch die Kammer zu pumpen, werden in üblicher Weise zu einer solchen Vorrichtung externe Pumpen benötigt. Dies bedeutet in dem Anwendungsbereich der Medizin, insbesondere bei Oxygenatoren, auf welchem die vorliegende Erfindung jedoch ausdrücklich nicht beschränkt ist, dass zusätzlich zu den bislang im Stand der Technik bekannten gattungsgemäßen Vorrichtungen eine externe Pumpe vorgesehen sein muss, mit der Blut aus dem Körper eines Patienten abgepumpt, durch die Vorrichtung gepumpt und sodann in den Körper des Patienten zurückgeleitet wird. Dabei kann es sich bei dem Patienten sowohl um einen menschlichen als auch einen tierischen Patienten handeln.

Durch den externen Einsatz von Pumpen ergibt sich das Problem, dass ein erheblicher Teil des gepumpten Blutvolumens von der Pumpe eingenommen wird, da diese für den Betrieb der Vorrichtung vollständig mit Blut gefüllt sein muss (großes extrakorporales Volumen = Verdünnung des Blutes). Daher sind gattungsgemäße Vorrichtungen gerade für den Einsatz bei Babys bzw. Frühgeborenen oder auch sehr kleinen Tieren kaum oder nur bedingt einsetzbar, da das Blutvolumen in diesen Fällen teilweise unter 100 Millilitern liegt, das Volumen von Pumpen jedoch üblicherweise in dieser Größenordnung oder sogar größer ist, so dass die Notwendigkeit besteht, bei der Behandlung von Babys oder Frühgeborenen bzw. kleinen Tieren zusätzliches Blutplasma oder Plasmaersatz oder Blutersatz bzw. Erythrozyten-Konzentrat einzusetzen, was zu einer starken Belastung des Organismus führen kann. WO2009/110652 offenbart eine Vorrichtung umfassend ein Gehäuse für Hohlfasern in dem ein Ballon angeordnet ist der die Kammern ringförmig umgibt.

DE102007010112 offenbart eine Vorrichtung für den Stoff- und/oder Energieaustausch zwischen zwei Medien welches ein Pumpelement als verformbares, insbesondere elastisch verformbares Element umfasst, welches insbesondere als verformbare, elastische verformbare Hohlfaser ausgebildet ist. Bei externen Pumpen, wie z.B. Schlauchpumpen ist auch ein weiteres Problem, gerade bei niedrigen (Blut-) Flussraten durch den sehr gleichmäßigen Lauf gegeben (schleichende Blutbewegung innerhalb des Oxygenators), da hierdurch ein sehr gleichmäßiger Blutstrom entsteht, der für die Sauerstoffanreicherung weniger günstig ist. Weiterhin können "Totwassergebiete" entstehen, und dadurch die Gefahr der Gerinnung- (und Komplement-) Aktivierung, und schließlich das Versagen des Oxygenators, z.B. durch Zuklotten des Blutes im Oxygenator. Dieses Problem kann in ähnlicher Weise auch bei anderen ersten und zweiten Medium bestehen.

Es ist daher die Aufgabe der Erfindung, eine Vorrichtung der eingangs genannten gattungsgemäßen Art wie in Anspruch 1 definiert bereitzustellen, bei denen mit möglichst geringem Volumen des ersten Medium, insbesondere möglichst geringem Blutvolumen eine ausreichende Pumpfunktion bereitgestellt werden kann, insbesondere um eine erfindungsgemäße Vorrichtung bei Babys und Frühgeborenen zu einer Anwendung bringen zu können. Weiterhin soll durch eine derart kleinbauende Ausführung einer erfindungsgemäßen Vorrichtung erzielt werden, dass diese auch in den Körper eines Patienten implantierbar ist. Weiterhin ist es die Aufgabe der Erfindung einen hoch effektiven Stoffaustausch zu erzielen.

Hier ist ergänzend darauf hinzuweisen, dass immer dann, wenn beispielhaft Blut als erstes Medium und Sauerstoff als zweites Medium bei der Beschreibung einer Ausführung der Vorrichtung genannt ist, die beschriebene Ausführung als offenbart gilt für jedes beliebige erste und zweite Medium.

Erfindungsgemäß wird diese Aufgabe dadurch gelöst, dass die Kammer zumindest in einem Bereich, der das Bündel von Hohlfasern vollständig umgibt, insbesondere in einer oder mehreren axial hintereinander liegenden Ebenen senkrecht zur Hohlfasererstreckung vollständig umgibt, als elastische Hülle ausgebildet ist, wobei um die elastische Hülle ein starres Gehäuse angeordnet ist, dessen Gehäuseinnenwand die Hülle in mehreren ersten Bereichen kontaktiert und zu dessen Gehäuseinnenwand die Hülle in wenigstens einem, bevorzugt mehreren zweiten Bereichen in Richtung der Hohlfasererstreckung kontaktfrei, insbesondere beabstandet ist, wobei der eine ausgebildete Hohlraum oder wenigstens einer der in den zweiten Bereichen zwischen Hülle und Gehäuse ausgebildeten Hohlräume durch die Gehäusewandung hindurch in einen Fluidanschluss mündet.

Der wesentliche Kerngedanke der Erfindung besteht hier darin, dass das interne Volumen der Kammer, welches hier durch die elastische Hülle umgrenzt ist, aufgrund der Elastizität der Hülle vergrößert und verringert werden kann. Dabei ist das minimale Volumen der Kammer dann erreicht, wenn die elastische Hülle aufgrund ihrer Elastizität, insbesondere einer eigenständigen Rückstellung oder durch Überdruck in der Kammer vollständig an dem Faserbündel anliegt. So kann das unerwünschte Auftreten von Randgängigkeit der Blutphase (der Spalt zwischen Gehäuse und Hohlfaserbündel) verhindert werden.

Das innere Volumen der durch die Hülle umgrenzten Kammer kann jedoch dadurch vergrößert werden, dass die elastische Hülle von dem Hohlfaserbündel abgehoben wird.

Dies kann gemäß der Erfindung nun dadurch erfolgen, dass sich zwischen der Hülle und dem Gehäuse, insbesondere dessen innerer Gehäusewandung in den mehreren vorgenannten zweiten Bereichen jeweils ein Hohlraum ergibt, der durch die Gehäusewandung hindurch in einen Fluidanschluss mündet, so dass durch diesen Fluidanschluss der Hohlraum mit einem Unterdruck gegenüber dem Innendruck in der Kammer bzw. innerhalb der elastischen Hülle versehen werden kann, so dass sich durch diesen Unterdruck in dem Hohlraum die elastische Hülle in den Hohlraum aufgrund ihrer elastischen Verformbarkeit hin verlagert wird und hierdurch das innere Kammervolumen vergrößert wird. Durch die Vergrößerung des Kammervolumens wird demnach das erste Medium in die Kammer hineingesaugt.

Hierbei kann es sich wie vorgenannt in einer bevorzugten Anwendung bei dem ersten Medium um Blut eines Patienten handeln.

Der Vorteil der erfindungsgemäßen Vorrichtung ergibt sich also maßgeblich dadurch, dass keinerlei Blut des Patienten benötigt wird, um zum Zweck der Pumpfunktion eine extern zum Blutkreislauf angeordnete Pumpe zunächst zu füllen, um deren Funktion zu gewährleisten. Dennoch kann die Vorrichtung auch mit einer externen Pumpe, z.B. einer Schlauchpumpe betrieben werden.

Das minimale Blutvolumen der Vorrichtung ist gegeben durch dasjenige Volumen, welches erreicht wird, wenn die elastische Hülle vollständig am Faserbündel anliegt, wohingegen das maximale Blutvolumen der Vorrichtung gegeben ist, wenn durch Unterdruckbeaufschlagung die elastische Hülle vollständig im Bereich des wenigstens einen zweiten Bereichs, der mit Unterdruck beaufschlagt ist, an die Gehäuseinnenwandung herangezogen wurde.

In einer möglichen Ausgestaltung, insbesondere einer solchen, bei der die Vorrichtung mit einer externen Pumpe betrieben wird, kann es vorgesehen sein, dass zwei erste Bereiche vorgesehen sind, wobei jeder am axialen Ende der Kammer angeordnet ist und die Kammer in Umfangrichtung umgibt, wobei zwischen diesen beiden ersten Bereichen ein zweiter Bereich angeordnet ist.

Die axialen Enden sind hierbei diejenigen Enden, welche die Kammer in der Längserstreckungsrichtung der Hohlfasern begrenzen. An diesen beiden einander gegenüberliegenden Enden kann das Gehäuse direkt unmittelbar oder über ein zwischengefügtes Element am der elastischen Hülle in Umfangsrichtung vollständig anliegen und so die Hülle ringförmig einfassen, so dass sich die Hülle an diesen Enden nicht von den Hohlfasern abheben kann.

Zwischen diesen beiden Enden ist die elastische Hülle anlagefrei, bzw. kontaktfrei zum Gehäuse. Es ist somit wenigstens ein Ringraum zwischen der elastischen Hülle und der Gehäuseinnenwandung zwischen den axialen Enden vorhanden. Dieser wenigstens eine, bevorzugt genau eine Ringraum bildet einen zweiten Bereich, der einen Fluidanschluß aufweist, um eine Druckänderung im Ringraum zu erzeugen und so das Volumen der Kammer zu ändern.

Bei dieser Ausführung kann eine Betriebsweise vorgesehen sein, bei der der wenigstens eine zweite Bereich bzw. der hierdurch ausgebildete Hohlraum / Ringraum über den Fluidanschluß mit periodischen Druckänderungen beaufschlagt wird, insbesondere wobei die Druckänderungen gewählt werden können im Bereich von z.B. 10 bis 120 oder 30 bis 60 Änderungen pro Minute, um ein Pumpen des ersten Mediums durch die Kammer zu bewirken.

Es kann auch vorgesehen sein, die Druckänderungen mit einer ersten niedrigen Anzahl von Änderungen pro Minute vorzusehen, z.B. im Bereich von 10 bis 120 oder 30 bis 60 Änderungen pro Minute zu wählen, wobei diesen ersten Druckänderungen zweite Druckänderung einer höheren Änderungsfrequenz und geringerer Amplitude, insbesondere 100 bis 300 Änderungen pro Minute überlagert werden, wobei durch die niederfrequenten Änderungen ein Pumpen des ersten Mediums durch die Kammer bewirkt wird und durch die höherfrequenten Änderungen eine Sekundärströmung / Durchmischung des ersten Mediums in der Kammer bewirkt wird.

Alternativ kann gerade mit dieser Ausführung der Vorrichtung die Druckänderung im Bereich von 100 bis 300 Änderungen pro Minute oder noch höher gewählt werden, um eine Sekundärströmung / Durchmischung des ersten Mediums in der Kammer zu bewirken, wobei das erste Medium durch die Kammer mittels einer separaten, insbesondere extern angeordneten Pumpe, z.B. einer Schlauchpumpe erfolgt. In dieser Variante wird die Vorrichtung in der Anwendung Blut / Sauerstoff somit lediglich als Oxigenator betrieben ohne eigene Pumpfunktion.

In einer anderen Ausführung der Vorrichtung kann es vorgesehen sein, dass die Gehäuseinnenwand die Hülle in mehreren ersten Bereichen, die jeweils in Richtung der Hohlfasern erstreckt sind, kontaktiert und die ersten und zweiten Bereiche in gleichmäßiger Winkelteilung das Bündel der Hohlfasern insbesondere in gleichmäßiger Winkelteilung umgeben.

Auch hier können die gleichen Verfahrensweisen eingesetzt werden, die zuvor beschrieben wurden, wobei es bevorzugt ist, durch die Vorrichtung selbst die Pumpfunktion zu realisieren, ggfs die Druckänderungen für die Pumpfunktion durch höherfrequente Druckänderungen mit geringerer Amplitude zu überlagern. Gemäß der Erfindung ist es vorgesehen, dass nicht nur in einem der zweiten Bereiche, sondern in mehreren der zweiten Bereiche die jeweils ausgebildeten Hohlräume durch die Gehäusewandung hindurch mit einem Fluidanschluss in Verbindung stehen, so dass in mehreren der insgesamt ausgebildeten zweiten Bereiche eine Unterdruckbeaufschlagung und damit eine Unterstützung der Pumpfunktion erzielt werden kann. Insbesondere ist es vorteilhaft, wenn für einen Anwender die Möglichkeit besteht, bei Vorhandensein von mehreren Fluidanschlüssen die Menge des gepumpten Volumens variieren zu können, nämlich dadurch, ob von den mehreren zur Verfügung stehenden Hohlräumen einer, zwei oder mehrere gleichzeitig mit Unterdruck beaufschlagt werden. So kann das maximal gepumpte Volumen variiert werden zwischen dem ein- und n-fachen Volumen eines jeden Hohlraums in einem zweiten Bereich, wenn insgesamt n Hohlräume eines zweiten Bereichs mit einem Fluidanschluss zur Unterdruckbeaufschlagung vorgesehen sind an der erfindungsgemäßen Vorrichtung. Dabei kann es bevorzugt vorgesehen sein, dass jeder Hohlraum gleiches Volumen hat.

Dabei wird es als bevorzugt angesehen, wenn die ersten und zweiten Bereiche in gleichmäßiger Winkelteilung das Bündel umgeben. Die Ausführung ist insoweit bevorzugt, als dass durch eine gleichmäßige Winkelteilung eine symmetrische Formgestaltung des Gehäuses verwendet werden kann, die technisch ohne großen Aufwand konstruiert werden kann. Hierbei kann jeweils ein ersten Bereich zwischen zwei zweiten Bereichen sowie in gleicher Weise ein zweiter Bereich zwischen zwei ersten Bereichen liegen. Über den Umfang können die ersten und zweiten Bereiche somit abwechselnd angeordnet sein.

Für eine solche bevorzugte Ausbildung kann es beispielsweise vorgesehen sein, dass das Bündel der Hohlfasern zusammen mit der umgebenden Hülle senkrecht oder zumindest quer (in einem Anströmungswinkel zwischen 0 und 90°) zur Hohlfasererstreckung einen im Wesentlichen kreisförmigen Außenquerschnitt aufweist.

In einer ersten möglichen Ausführung kann das umgebende Gehäuse an diesen Querschnitt angepasst sein und dabei in der inneren Gehäusewand, bevorzugt in gleichmäßiger Winkelteilung mehrere Ausnehmungen bzw. Rücksprünge aufweisen, so dass in diesen Bereichen die Hülle nicht an der inneren Gehäusewand anliegt. Solche Rücksprünge oder Ausnehmung können dabei eine Längserstreckung in Richtung der Hohlfasererstreckung aufweisen. Durch jeden dieser rückspringenden oder ausgenommenen Bereiche wird ein Hohlraum umgrenzt, der in Richtung zum Bündel durch die Hülle begrenzt ist und in den ein Fluidanschluß durch die Gehäusewandung hindurch münden kann, insbesondere um den Hohlraum mit Unterdruck und/oder Überdruck zu beaufschlagen und so die elastische Hülle in den Hohlraum hinein zu verlagern, insbesondere hinein zu saugen.

In einer weiteren Ausführung kann das Gehäuse einen im Wesentlichen mehreckigen freien Innenquerschnitt aufweist, wobei zwischen den Eckbereichen der Gehäuseinnenwand und der Hülle in Richtung der Hohlfasererstreckung zweite Bereiche ausgebildet sind. Dabei wird von einem mehreckigen Querschnitt des Gehäuses im Innenbereich im Sinne der Erfindung auch dann gesprochen, wenn die Eckbereiche beispielsweise gerundet ausgeführt sind, somit also der mehreckige Querschnitt nicht im eigentlichen mathematischen Sinne als mehreckig zu verstehen ist. Wesentlich ist es hier, dass sich zwischen den Eckbereichen bei einer mehreckigen Querschnittsform ebene Flächenbereiche der Gehäuseinnenwand ergeben, die in den Eckbereichen ineinander übergehen.

In bevorzugter Ausgestaltung der Vorrichtung kann es bei allen möglichen Gehäusevarianten vorgesehen sein, dass in zwei gegenüberliegenden zweiten Bereichen in dem Gehäuse Ausnehmungen / Durchbrüche vorgesehen sind, durch welche an die Hülle angeformte Anschlüsse zur Zu- und Abfuhr des ersten Mediums durch diese Gehäusewandung hindurchgeführt sind. Hierbei kann in wenigstens einem der Anschlüsse ein Einwege-Ventil oder ein aktives Ventile angeordnet sein, welches einen Fluß des ersten Mediums in nur einer definierten Richtung zulässt und somit wie ein Rückschlagventil wirkt oder einem solchen entspricht.

Beispielsweise kann an die Hülle, die aus einem elastischen Material gebildet ist, wie z.B. Silikon, Latex oder sonstiges blutverträgliches Material, einstückig jeweils für den Zu- und Abfluss ein Schlauchstück angeformt sein, wobei diese beiden Schlauchstücke an der Hülle einander gegenüberliegend angeordnet sind, so dass sicher gestellt ist, dass das zu- bzw. abgeführte Blut diametral durch den Innenquerschnitt der Hülle in Richtung quer, bevorzugt senkrecht zur Fasererstreckung an den Hohlfasern vorbeigeführt wird.

Bei der Ausführung mit einem n-eckigen Innenquerschnitt des Gehäuses, welches die Hülle umgibt, können demnach diese Zu- und Abfuhranschlüsse für das erste Medium im Bereich von zwei gegenüberliegenden Ecken des Querschnittes angeordnet sei, so dass insgesamt (n-2) zweite Bereiche zwischen Hülle und innerer Gehäusewandung verbleiben, an denen Hohlräume ausgebildet sind, insbesondere die jeweils durch das Gehäuse mit Anschlüssen zur Unterdruckbeaufschlagung versehen sein können.

So können bei einer erfindungsgemäßen Vorrichtung mit einem n eckigen Innenquerschnitt des die Hülle umgebenden Gehäuses (n-2) Abstufungen des mit einem Pumphub geförderten Volumens erzielt werden, wobei jede Volumenstufe demjenigen Volumen entspricht, welches in einem der zweiten Bereiche zwischen der Gehäusewand und der Hülle eingeschlossen ist.

So kann es in bevorzugter Ausführung bei n-eckiger Gehäuseausführung sowie auch Gehäusen mit mehreren Ausnehmungen / Rücksprüngen im zweiten Bereich vorgesehen sein, dass in zwei gegenüberliegenden, bevorzugt allen verbleibenden zweiten Bereichen, die zwischen Hülle und Gehäuse ausgebildeten Hohlräume durch die Gehäusewandung hindurch mit einem Fluidanschluss versehen sind und mit einem Unterdruck, z.B. durch eine externe Unterdruckpumpeinheit beaufschlagbar sind.

Die bevorzugte Ausführung kann dabei so gewählt sein, dass, insbesondere zwischen der Gehäuseinnenwandung und der Hülle, eine gerade Anzahl von zweiten Bereichen ausgebildet ist, insbesondere wofür der Innenquerschnitt des Gehäuses senkrecht zur Richtung der Hohlfasererstreckung mehreckig ist mit einer geraden Anzahl von Ecken. Hierdurch ergibt sich gerade eine Symmetrie um die Mittenachse in Richtung der Hohlfasererstreckung, bei der jeweils diametral einander gegenüberliegende zweite Bereiche existieren, von denen zwei gegenüberliegende Bereiche für die Zu- und Abfuhr des ersten Mediums verwendet werden können und die verbleibenden jeweils gegenüberliegenden Bereiche zur Unterdruckbeaufschlagung, um die gewünschte Pumpfunktion zu erzielen.

Um sicherzustellen, dass in den ersten Bereichen die Hülle die Gehäuseinnenwand dicht kontaktiert und somit die Einzelvolumina in den jeweiligen zweiten Bereichen voneinander getrennt sind, kann es vorgesehen sein, dass in diesen ersten Bereichen die Hülle fest mit der Gehäuseinnenwand verbunden / verbindbar ist.

Dies kann in einer bevorzugten Ausführung dadurch erzielt werden, dass in den ersten Bereichen sich auf der Hülle in Richtung der Hohlfasern erstreckende Vorsprünge angeordnet sind, die jeweils in einer sich in der Gehäuseinnenwand in Richtung der Hohlfasern erstreckende Nut insbesondere formschlüssig einliegen bzw. einlegbar sind. Durch den Formschluss zwischen Vorsprung und Nut entlang der Erstreckung in Richtung der Hohlfasern wird eine Dichtung zwischen der Außenseite der Hülle und der Innenseite der inneren Gehäusewandung erzielt, so dass zwischen einander angrenzenden zweiten Bereichen keinerlei Überströmen von Fluid möglich ist und somit jeder in einem zweiten Bereich gebildete Hohlraum zwischen Gehäuseinnenwand und Hülle separat und individuell mit Unterdruck beaufschlagbar ist.

Für eine Montage kann es dabei vorgesehen sein, dass jeder der vorgenannten, sich in Hohlfaserrichtung erstreckenden Vorsprünge in Richtung der Hohlfasererstreckung in die Nut an der Gehäuseinnenwand eingeschoben wird, insbesondere bei einer Montage, bei welcher in axialer Richtung die Gesamtanordnung aus Bündel und Hülle in das starre Gehäuse geschoben wird.

Dabei kann die Ausbildung der Nut derart sein, dass die Nutöffnung in Richtung zur Hülle eine geringere Breite aufweist als das Nutinnere, so dass ein entsprechend ausgebildeter Vorsprung auf der Hüllenaußenseite nahe an der Hüllenoberfläche eine geringere Breite aufweist als in einer weiteren Entfernung zur Hüllenoberfläche. Beispielsweise kann der Nutquerschnitt und der Vorsprungsquerschnitt senkrecht zur Erstreckung der Hohlfaserrichtung kreisförmig oder auch dreieckig ausgeführt sein, so dass ein in die entsprechende Nut eingeschobener Vorsprung in radialer Richtung sicher in der Nut gehalten ist und in dieser gedichtet einliegt.

In einer weiterhin bevorzugten Weiterbildung der erfindungsgemäßen Vorrichtung kann es ergänzend vorgesehen sein, dass das Bündel der Hohlfasern auf einen Kern gewickelt ist. Ein solcher Kern kann zum einen zur Stabilisierung des Hohlfaserbündels vorgesehen sein und zum anderen in einer erfindungsgemäßen Vorrichtung hohl ausgebildet sein, wobei die äußere, zum Bündel weisende Kernwandung zumindest bereichsweise Durchbrüche zum hohlen Kerninneren aufweist, die von einer flexiblen Membran verschlossen/überdeckt sind und wobei das Kerninnere einen Fluidanschluss aufweist, insbesondere über den das Kerninnere in gleicher Weise mit Unterdruck beaufschlagbar ist, wie die einzelnen zuvor genannten Hohlräume in den jeweiligen zweiten Bereichen zwischen Hülle und Gehäuseinnenwand.

Es besteht bei dieser Ausführung demnach ebenso die Möglichkeit, die Pumpfunktion durch eine Unterdruckbeaufschlagung und/oder Überdruckbeaufschlagung des Kerninneren zu unterstützen, wodurch eine Verlagerung der flexiblen Membran, die die Kerndurchbrüche verschließt, in das Innere des Kernes erfolgt, so dass hierdurch das effektive Volumen der Vorrichtung ebenso vergrößert wird.

Hierbei kann es vorgesehen sein, dass der Kern senkrecht zur Richtung der Hohlfasererstreckung einen mehreckigen Außenquerschnitt aufweist, wobei Durchbrüche in den planen Flächenbereichen zwischen den in Hohlfaserrichtung verlaufenden Kanten des Kerns angeordnet sind. Hierbei kann bevorzugt die Anzahl der Ecken des Kerns der Anzahl der Ecken des Gehäuses entsprechen.

Bei einer solchen Ausführung eines hohlen Kerns kann unabhängig von dessen Außenquerschnitt, der gegebenenfalls auch rund ausgeführt sein kann, der Kern eine flexible elastische Hülle tragen, die die Durchbrüche des Kerns überdeckt. Eine solche Hülle kann z.B. durch einen Schlauch gebildet sein, der dicht auf der Kernoberfläche aufliegt.

Ein bevorzugtes Ausführungsbeispiel der Erfindung ist in den nachfolgenden Figuren beschrieben. Es zeigen:
- Figur 1: eine Schnittansicht der Kammer zur Führung des ersten Mediums senkrecht zur Richtung der Hohlfaser
- Figur 2: eine perspektivische Ansicht derselben Kammer
- Figur 3: das Gehäuse einer erfindungsgemäßen Vorrichtung mit mehreckigem Innenquerschnitt
- Figur 4: eine Schnittansicht senkrecht zur Hohlfasererstreckung der gesamten erfindungsgemäßen Vorrichtung
- Figur 5: eine perspektivische Ansicht der gesamten erfindungsgemäßen Vorrichtung
- Figur 6: eine Schnittansicht senkrecht zur Hohlfasererstreckung mit einem zusätzlich hohl ausgebildeten Kernbereich zur Unterstützung der Pumpfunktion
- Figur 7: Die Dehnung der Hülle innerhalb eines zweiten Bereiches
- Figur 8: den durch Ansteuerung erzielbaren zeitlichen Verlauf der Dehnung
- Figur 9: eine Ausführung mit axialer endseitiger Kontaktierung in zwei ersten Bereichen und dazwischen angeordnetem zweiten Bereich / Ringraum

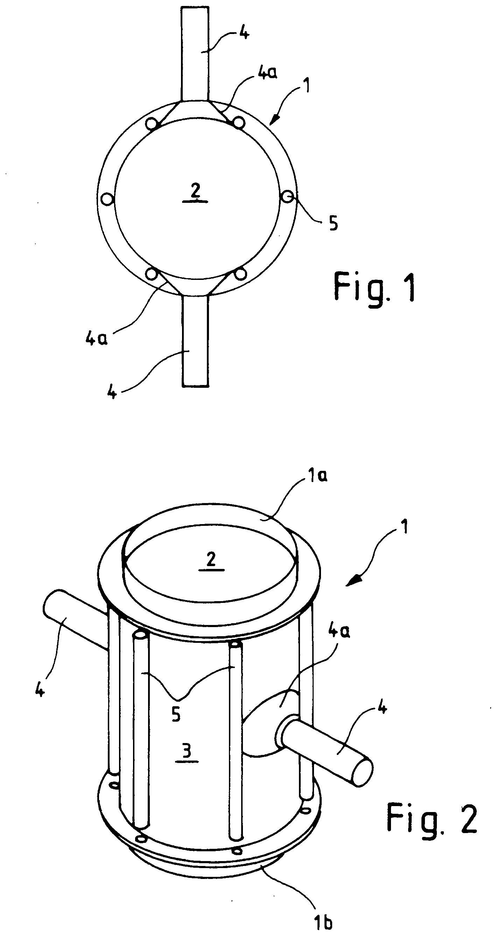

Die Figuren 1 und 2 zeigen in zwei verschiedenen Ansichten die Kammer 1 einer erfindungsgemäßen Vorrichtung, in welcher ein Bündel von Hohlfasern angeordnet ist, welches in den Figuren 1 und 2 nicht separat dargestellt ist.

Bezogen auf die Figur 1 befindet sich das Bündel der Hohlfasern im inneren Bereich 2 der Kammer 1, wobei bezogen auf diese Figur 1 die Erstreckung der Hohlfasern senkrecht zur Papierebene erfolgt.

Unter Bezugnahme auf die Figuren 1 und 2 weist die Kammer 1 eine im Wesentlichen zylindrische Form auf mit einem im Wesentlichen kreisrunden Querschnitt senkrecht zur Hohlfasererstreckung. Die Kammer 1 ist hier gegebenenfalls bis auf die axialen Endbereiche 1a und 1b durch eine sich zwischen diesen axialen Endbereichen erstreckende elastische Hülle 3 ausgebildet, die aufgrund ihrer Elastizität und damit ihrer Tendenz, sich maximal zusammenzuziehen, außen an dem aufgenommenen Hohlfaserbündel anliegt. Diese Hülle 3 umgibt das Bündel in radialer Richtung vollständig. Die axialen Enden 1a und 1b können zu einem späteren Zeitpunkt an der Hülle 3 angebracht, z.B. gedichtet angeformt werden, z.B. stoffschlüssig, beispielsweise nachdem die Hülle 3 mit dem innenliegenden Bündel in ein Gehäuse eingesetzt wurde.

Die axialen Enden des Hohlfaserbündels, welches in den Figuren 1 und 2 nicht dargestellt ist, enden gedichtet in der Querschnittsebene der beiden axialen Endbereiche 1a und 1b der Kammer 1, so dass durch diese axialen Endbereiche 1a und 1b die einzelnen Hohlfasern mit einem Fluid beaufschlagt werden können, welches sodann in der Richtung der Erstreckung der Hohlfasern durch diese hindurchgeleitet wird.

Erkennbar ist hier weiterhin, dass an die elastische Hülle 3 in einer Richtung senkrecht zur Hohlfasererstreckung Anschlüsse 4 für die Zu- und Abführung des ersten Mediums vorgesehen sind. Hier können diese Anschlüsse 4 beispielsweise als Schlauchanschlüsse ausgebildet sein, die einstückig an die flexible Hülle 3 angeformt sind. Demnach können die Anschlüsse 4 aus dem gleichen elastischen Material gebildet sein, wie die Hülle 3 selbst.

Bezugnehmend auf die Figuren 1 und 2 ist somit erkennbar, dass in einer Richtung senkrecht zur Hohlfasererstreckung das erste Medium durch die hier dargestellte Kammer 1 hindurchgeführt werden kann. Innerhalb wenigstens eines der Anschlüsse 4, insbesondere im Bereich nahe der Hülle 3, kann zur Definition einer Fließrichtung beispielsweise ein Einwegeventil vorgesehen sein. Der flexible Übergangsbereich 4a zwischen einem Anschluss 4 und der Hülle 3 kann als kollapsibles Reservoir dienen.

Es ist hier weiterhin in den Figuren 1 und 2 dargestellt, dass auf der Oberfläche der Hülle 3 in einer Richtung parallel zur Hohlfasererstreckung mehrere Vorsprünge 5 vorgesehen sind, die im vorliegenden Fall mit einer gleichmäßigen Winkelteilung die Hülle 3 umgeben. Die Vorsprünge 5 erstrecken sich in bevorzugter Ausführung über die gesamte axiale Länge zwischen den Endbereichen 1a und 1b.

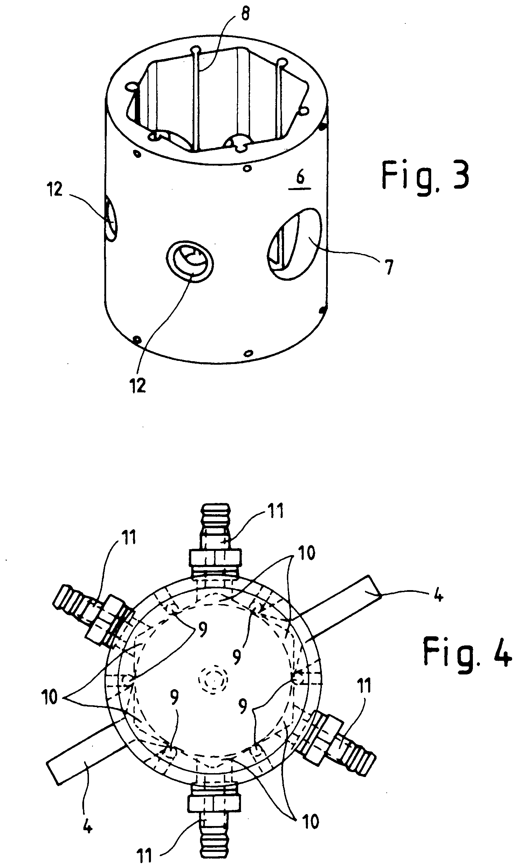

Die Figur 3 zeigt in alleiniger Darstellung das Gehäuse einer erfindungsgemäßen Vorrichtung, welches dazu dient, die Kammer 1 aus der elastischen Hülle 3 mit dem darin angeordneten Hohlfaserbündel, wie sie in den Figuren 1 und 2 dargestellt ist, in ihrem Inneren aufzunehmen. Dabei können die Anschlüsse 4 zum Zuführen und Abführen des ersten Mediums durch Ausnehmungen 7 des Gehäuses 6 geführt werden, die einander diametral gegenüberliegen.

Die Kammer 1, wie sie in den Figuren 1 und 2 dargestellt ist, kann hier in Richtung der Hohlfasererstreckung in den inneren, im Querschnitt mehreckigen Aufnahmebereich des Gehäuses 6 eingeschoben werden, wobei es vorgesehen ist, dass die Vorsprünge 5 in die Nuten 8 in der Innenwandung des Gehäuses 6 eingeschoben werden. Hierfür haben die Nute 8 und die Vorsprünge 5 zueinander korrespondierende Querschnitte senkrecht zur Hohlfasererstreckung.

Bezogen auf die Figur 4 ergibt sich eine Gesamtanordnung, bei welcher erkennbar ist, dass in ersten Bereichen 9 die Hülle 3 gedichtet an der im Querschnitt hier sechseckigen Gehäuseinnenwandung anliegt, wobei hier die Dichtung zwischen Hülle und Gehäuseinnenwand durch die formschlüssige Verbindung zwischen den Vorsprüngen 5 und den Nuten 8 erzielt wird.

Zwischen den ersten Bereichen 9 sind in ebenso gleichmäßiger Winkelteilung zweite Bereiche 10 gebildet, in denen die an den Hohlfasern anliegende elastische Hülle 3 zur Gehäuseinnenwandung beabstandet ist, so dass sich zwischen dem kreisförmigen Außenquerschnitt der Hülle und dem n-eckigen, hier sechseckigen Innenquerschnitt des Gehäuses 6, in diesen zweiten Bereichen 10 Hohlräume ergeben. Die einzelnen, in den zweiten Bereichen 10 ausgebildeten Hohlräume sind hierbei voneinander durch die formschlüssige dichte Verbindung von Vorsprung 5 und Nut 8 gedichtet voneinander getrennt.

Hierbei zeigt die Figur 4, dass in zwei gegenüberliegenden zweiten Bereichen die gegenüberliegenden Ausnehmungen 7 des Gehäuses 6 angeordnet sind, durch welches die Anschlüsse 4 für die Zu- und Abfuhr des ersten Mediums geführt sind.

In allen verbleibenden zweiten Bereichen 10 münden die in diesen zweiten Bereichen 10 gebildeten Hohlräume in Fluidanschlüsse 11, durch welche jeder der einzelnen Hohlräume individuell oder auch mehrere gemeinsam und gleichzeitig mit Unterdruck und/oder Überdruck beaufschlagt werden können, wodurch die Möglichkeit besteht, die Hülle 3, die das Hohlfaserbündel umgibt, in dem jeweils mit Unterdruck beaufschlagten zweiten Bereich 10 in die Richtung der Gehäuseinnenwand zu verlagern und dadurch das innere Volumen der Kammer 1 zu vergrößern. Durch eine solche Vergrößerung wird Blut durch den Einlassanschluss 4 in die Kammer hineingesaugt, wobei durch wenigstens ein Einwegeventil im Ein- bzw. Auslass 4 sichergestellt wird, dass eine Blutförderung in eine vorgegebene definierte Richtung erfolgt.

Wird die Unterdruckbeaufschlagung abgestellt oder sogar eine Überdruckbeaufschlagung vorgenommen, so stellt sich die Hülle 3 wieder in ihre Ausgangslage zurück und liegt an dem Faserbündel außen an, wodurch das innere Volumen der Kammer 3 verkleinert und das erste Medium durch einen der Anschlüsse 4 aus der Kammer herausgepresst wird.

Die hier dargestellten Fluidanschlüsse 11 können in dafür vorgesehenen Ausnehmungen in dem Gehäuse 6 angeordnet sein, beispielsweise können diese gedichtet in diese Ausnehmungen 12 eingeschraubt sein.

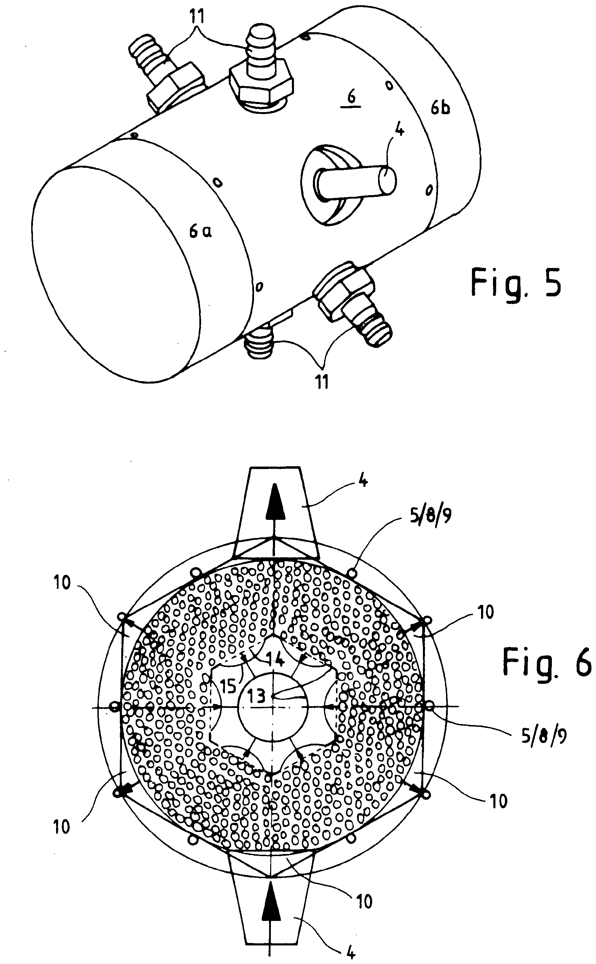

Eine perspektivische Ansicht der Figur 4 ist in der Figur 5 dargestellt, wobei erkennbar ist, dass an das Gehäuse 6 zu beiden axialen Seiten Kappen 6a und 6b befestigt sind, über welche die Beaufschlagung des in dem Gehäuse 6 angeordneten Hohlfaserbündels mit dem zweiten Medium erfolgen kann. Die diesbezüglichen Fluidanschlüsse sind in der Figur 5 nicht dargestellt.

Ersichtlich ergibt sich hier der weitere Vorteil, dass die Kammer 1 und damit das Faserbündel mit der umgebenden elastischen Hülle 3 aus dem Gehäuse 6 nach einer Benutzung entfernt und eine neue Kammer 1 eingesetzt werden kann. Demnach kann ein großer Teil einer erfindungsgemäßen Vorrichtung wieder verwendet werden, wobei nur der das erste Medium führende Teil aus hygienischen Gründen, insbesondere bei der Anwendung der Vorrichtung, im medizinischen Bereich ausgetauscht werden kann.

Die Figur 6 zeigt im Wesentlichen einen zur Figur 4 entsprechenden Querschnitt unter weiterer Andeutung des Faserbündels, wobei dieses Faserbündel im vorliegenden Fall auf einen inneren Kern 13 aufgewickelt ist, der in dieser jedoch nicht beschränkenden Ausführungsform ebenfalls n-eckig (n=6) ausgeführt ist. Die zwischen den einzelnen äußeren Eckbereichen des Kerns gegebenen ebenen Flächenbereiche weisen Durchbrüche 14 auf, die von einer elastischen Membran 15 verschlossen bzw. überdeckt sind.

In einer Ausführung kann sich diese Membran 15 als Hülle bzw. als Schlauch über die gesamte axiale Länge des Kerns 13 erstrecken und befindet sich demnach zwischen Kern 13 und Faserbündel. Der innere hohle Bereich des Kerns 13 kann durch einen hier nicht separat dargestellten Anschluss ebenfalls mit Unterdruck und/oder Überdruck beaufschlagt werden, wodurch eine Membran 15, wie hier dargestellt, in den inneren hohlen Bereich des Kerns 13 verlagert werden kann und in gleicher Weise das Kammervolumen vergrößert wie eine Unterdruckbeaufschlagung über die Anschlüsse 11.

Die Ausgestaltung eines hohlen Kerns mit Durchbrüchen, die von einer elastischen Membran oder Hülle verschlossen sind, kann demnach in gleicher Weise zur Unterstützung der Pumpfunktion verwendet werden wie die Unterdruckbeaufschlagung über die Anschlüsse 11, so dass durch eine entsprechende Auswahl der Anzahl von Anschlüssen, die für die Unterdruckbeaufschlagung verwendet werden, das in einem Hub gepumpte Volumen bestimmt werden kann. Darüber hinaus kann es vorgesehen sein, eine phasenverschobene Unterdruckansteuerung der einzelnen Anschlüsse vorzunehmen und so nacheinander ein vordefiniertes Blutvolumen durch zeitlich nacheinander erfolgende Unterdruckansteuerung der Anschlüsse 11 bzw. des inneren Kerns vorzunehmen.

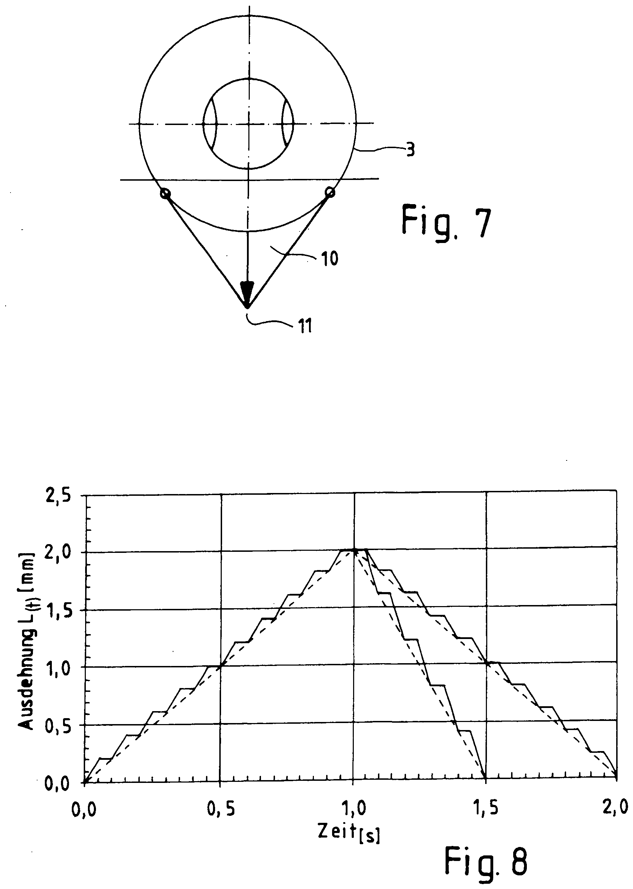

Die Figur 7 zeigt in Verbindung mit der Figur 8 die Möglichkeiten, den zeitlichen Verlauf der Auslenkung L der Hülle 3 am Ort der zweiten Bereiche 10 auf eine Gehäuseecke / -kante zu variabel zu steuern. Der zeitliche Verlauf in der Ausdehnung L der Hülle 3 an diesem Ort in die Richtung der Anschlüsse 11 ist ein wichtiger Parameter zur Erzeugung der Strömung des ersten Mediums, hier insbesondere von Blut im Volumen der Kammer 1 und beeinflusst damit wesentlich, insbesondere bei einer Anwendung als Oxygenator den Stofftransfer zwischen den beiden Medien, hier insbesondere vom Gas zum Blut.

Hier kann es vorgesehen sein, so wie es die Figur 8 zeigt, dass die Zeit zum Saugen und somit zur Erzeugung des Unterdrucks im Hohlraum am Bereich 10 verschieden eingestellt wird gegenüber der Zeit zum Drücken bzw. um den Unterdruck zu verringern, um die Rückstellung der Membran 3 und die Anlage derselben an den Hohlfasern zu bewirken. Der zeitliche Verlauf der Membranauslenkung und damit die Volumenänderung kann dabei sowohl beim Saugen oder Drücken linear eingestellt sein, wie es die Figur 8 anhand der gestrichelten Linien zeigt, ebenso wie mit einem hier im Wesentlichen sägezahnförmigen Verlauf mit unterschiedlichen, wahlweise einstellbaren Flankensteilheiten. Diese verschiedenen Einstellungen können insbesondere dadurch vorgenommen werden, dass die Applikation des Unterdrucks / Überdrucks über die Anschlüsse 11 bzw. auch den inneren Anschluss bei dem Vorsehen eines hohlen Kerns variiert wird. Es lässt sich somit gezielt Einfluss nehmen auf die Art und den zeitlichen Verlauf der Strömung des ersten Mediums, hier insbesondere von Blut.

Ein weiterer wesentlicher Vorteil der hier dargestellten erfindungsgemäßen Vorrichtung ist der, dass diese besonders kleinbauend ausgeführt werden kann und ein geringes Füllvolumen aufweisen kann, welches auch für die Anwendung bei Babys und Frühgeborenen oder kleinen Tieren ermöglicht. Beispielsweise kann das Füllvolumen kleiner 100 Milliliter, bevorzugt kleiner 50 Milliliter, besonders bevorzugt kleiner 30 Milliliter gewählt sein, insbesondere um bei Babys und Frühgeborenen eine extrakorporale Lungenunterstützung zu bieten.

Insbesondere bezogen auf die Figuren 1 und 2 kann es hier vorgesehen sein, dass für die Hohlfasern und/oder die Hülle 3 Silikon als Material verwendet wird. Hier kann es vorgesehen sein, die Hülle, die Anschlüsse 4 sowie die in axialer Richtung liegenden Anschlüsse bzw. Abdichtungen zum Hohlfaserbündel aus einem Guss herzustellen. Es kann vorgesehen sein, Deckel 6a und Boden 6b des Gehäusekörpers 6 gedichtet über die axialen Enden 1a und 1b der inneren Kammer 1 zu ziehen, so dass auch ein untereinander Verkleben der einzelnen Komponenten entbehrlich wird und dies die Möglichkeit zur Wiederverwendung der äußeren Gehäusekomponenten erschließt.

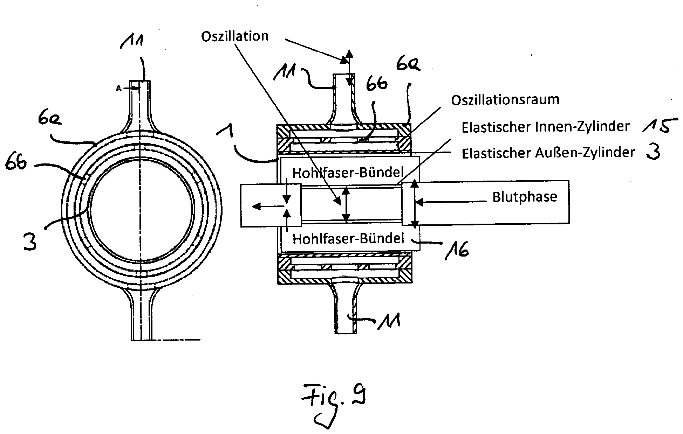

Die Figur 9 zeigt eine Ausführung der Vorrichtung, bei der die elastische Hülle 3 an den beiden axialen Enden des Hohlfaserbündels 16 ringförmig vom hier zweiteilig aufgebauten Gehäuse kontaktiert wird. Diese axialen Enden bilden somit zwei erste Bereiche.

Zwischen diesen axialen Enden ist das Gehäuse kontaktfrei zur elastischen Hülle 3, so dass diese durch eine Unterdruckbeaufschlagung durch den Fluidanschluß 11, der in der zweite Bereich / ringförmigen Hohlraum mündet, vom Faserbündel 16 abgehoben werden kann. Hierdurch ändert sich das Volumen der durch die Hülle 3 umschlossenen Kammer 1.

Das die Hülle 3 umgebende Gehäuse ist hier beispielsweise in radialer Richtung zweiteilig aufgebaut, aus den Bauteilen 6a und 6b. 6b bildet ein zylindrisches Element mit Durchbrechungen /Öffnungen. Es kann einen Anschlag für die Hülle 3 bilden, so dass die Volumenvergrößerung begrenzt ist, z.B. wenn das Gesamtvolumen zwischen Gehäuse und Hülle 3 größer ist als benötigt. Auch wird hierdurch sichergestellt, dass die Hülle den Fluidanschluss 11 nicht verschließen kann, wenn sich diese aufbläht.

Es kann bei dieser Ausbildung der Vorrichtung vorgesehen sein, dass das Blut oder ein beliebiges Medium mittels hier nicht gezeigter Anschlüsse durch die Kammer 1 gepumpt wird, z.B. mit einer externen nicht gezeigten Pumpe, wie beispielsweise einer Schlauchpumpe. In diesem Fall übernimmt die Vorrichtung keine Pumpfunktion. Das Blut kann dabei wie zuvor beschrieben zwischen den Anschlüssen z.B. quer zu dem Hohlfaserbündel strömen, also im wesentlichen radial zur Hohlfasererstreckung. Es ist aber auch möglich das Blut in Hohlfaserrichtung strömen zu lassen.

Durch wenigstens einen der Fluidanschlüsse 11 wird hier ergänzend im Betrieb der Vorrichtung eine Druckoszillation / -pulsation aufgeschaltet, wobei die Druckschwankungen z.B. im Bereich von 100 bis 300 Schwankungen pro Minute liegen können, aber auch höher.

Die Druckschwankungen übertragen sich durch die elastische Hülle auf das gepumpte Blut, das Sekundärströmungen hervorruft und eine verbesserte Blutdurchmischung, bzw. Kontaktierung mit den permeablen Hohlfasern bewirkt und somit einen Stoffaustausch verbessert. Dies gilt unabhängig für jede Paarung von zwei stoffaustauschenden Medien und nicht nur für Blut / Sauerstoff.

## Patentansprüche

1. Vorrichtung für den Stoff- und /oder Energieaustausch zwischen zwei Medien, insbesondere zwischen Blut und einem Gas / Gasgemisch, mit einer von dem ersten Medium, insbesondere von Blut durchströmbaren Kammer (1), in der quer zur Durchströmungsrichtung des ersten Mediums ein Bündel von stoff- und/oder energiepermeablen Hohlfasern angeordnet ist, die von dem zweiten Medium durchströmbar und vom ersten Medium umströmbar sind, wobei die Kammer (1) zumindest in einem Bereich, der das Bündel vollständig umgibt, als elastische Hülle (3) ausgebildet ist und um die elastische Hülle (3) ein starres Gehäuse (6) angeordnet ist, dessen Gehäuseinnenwand die Hülle (3) in mehreren ersten Bereichen (9) kontaktiert **dadurch gekennzeichnet, dass** die mehreren ersten Bereiche (9) jeweils in Richtung der Hohlfasern erstreckt sind und die Hülle (3) zu der Gehäuseinnenwand in mehreren zweiten Bereichen (10) in Richtung der Hohlfasererstreckung kontaktfrei, insbesondere beabstandet ist und die ersten und zweiten Bereiche (9,10) das Bündel, insbesondere in gleichmäßiger Winkelteilung umgeben, wobei mehrere der in den zweiten Bereichen (10) zwischen Hülle (3) und Gehäuse (6) ausgebildeten mehreren Hohlräume durch die Gehäusewandung hindurch jeweils in einen Fluidanschluß (11) münden, durch den der jeweilige Hohlraum mit einem Unterdruck gegenüber dem Innendruck in der Kammer (1) versehbar ist und die elastische Hülle aufgrund ihrer elastischen Verformbarkeit in den Hohlraum verlagerbar und hierdurch das Kammervolumen vergrößerbar ist.

2. Vorrichtung nach Anspruch 1 wobei das Bündel mit der Hülle (3) einen im Wesentlichen kreisförmigen Außenquerschnitt ausweist und das umgebende Gehäuse (6) an diesen Querschnitt, insbesondere formgleich, angepasst ist und in der inneren Gehäusewand, bevorzugt in gleichmäßiger Winkelteilung, mehrere Ausnehmungen bzw. Rücksprünge aufweist, die zweite Bereiche (10) ausbilden.

3. Vorrichtung nach Anspruch 1, wobei das Bündel mit der Hülle (3) einen im Wesentlichen kreisförmigen Außenquerschnitt ausweist und das Gehäuse (6) einen im Wesentlichen mehreckigen Innenquerschnitt aufweist, wobei zwischen den Eckbereichen der Gehäuseinnenwand und der Hülle in Richtung der Hohlfasererstreckung zweite Bereiche ausgebildet sind.

4. Vorrichtung nach einem der vorherigen Ansprüche, wobei in zwei gegenüberliegenden zweiten Bereichen (10) an die Hülle (3) angeformte Anschlüsse (4) zur Zu- und Abfuhr des ersten Mediums durch Ausnehmungen (7) im Gehäuse (6) durch die Gehäusewandung hindurchgeführt sind, insbesondere wobei in wenigstens einem der Anschlüsse (4) ein Einwege-Ventil oder ein aktives Ventil, insbesondere das von außen den Schlauch zusammendrückt und loslässt, angeordnet ist.

5. Vorrichtung nach Anspruch 4, wobei in zwei gegenüberliegenden, bevorzugt allen verbleibenden zweiten Bereichen (10) die zwischen Hülle (3) und Gehäuse (6) ausgebildeten Hohlräume durch die Gehäusewandung hindurch in einen Fluidanschluß (11) münden, insbesondere der mit Unterdruck und/oder Überdruck beaufschlagbar ist.

6. Vorrichtung nach einem der vorherigen Ansprüche, wobei zwischen der Gehäuseinnenwandung und der Hülle (3) eine gerade Anzahl von zweiten Bereichen (10) ausgebildet ist, insbesondere der Innenquerschnitt des Gehäuses senkrecht zur Richtung der Hohlfasererstreckung mehreckig ist mit einer geraden Anzahl von Ecken.

7. Vorrichtung nach einem der vorherigen Ansprüche, wobei in den ersten Bereichen (9) sich auf der Hülle (3) in Richtung der Hohlfasern erstreckende Vorsprünge (5) angeordnet sind, die jeweils in einer sich in der Gehäuseinnenwand in Richtung der Hohlfasern erstreckende Nut (8) insbesondere formschlüssig einliegen.

8. Vorrichtung nach einem der vorherigen Ansprüche, wobei das Bündel der Hohlfasern auf einen Kern (13) gewickelt ist.

9. Vorrichtung nach Anspruch 8, wobei der Kern (13) hohl ausgebildet ist, wobei die äußere zum Bündel weisende Kernwandung zumindest bereichsweise Durchbrüche (14) zum hohlen Kerninneren aufweist, die von einer flexiblen Membran (15) verschlossen / überdeckt sind und wobei das Kerninnere einen Fluidanschluß aufweist, insbesondere über den das Kerninnere mit Unterdruck und/oder Überdruck beaufschlagbar ist.

10. Vorrichtung nach Anspruch 8 oder 9, wobei der Kern (13) senkrecht zur Richtung der Hohlfasererstreckung einen mehreckigen Außenquerschnitt aufweist, wobei Durchbrüche (14) in den planen Flächenbereichen zwischen den in Hohlfaserrichtung verlaufenden Kanten des Kerns (13) angeordnet sind, insbesondere wobei der Kern (13) im Querschnitt eine zum Gehäuse gleiche Eckenanzahl hat.

11. Vorrichtung nach einem der Ansprüche 8 bis 10, wobei der Kern eine flexible elastische Hülle (15) trägt.

## Claims

1. Apparatus for mass and/or energy transfer between two media, in particular between blood and a gas/ gas mixture, with a chamber (1) through which the first medium, in particular blood, can flow and in which, transversely with respect to the direction of flow of the first medium, a bundle of hollow fibres permeable to mass and/or energy is arranged through which the second medium can flow and around which the first medium can flow, wherein the chamber (1) is designed as an elastic shell (3), at least in a region which completely surrounds the bundle, and a rigid housing (6) is arranged around the elastic shell (3), the inner wall of which housing contacts the shell (3) in a plurality of first regions (9), **characterized in that** the plurality of first regions (9) each extend in the direction of the hollow fibres, and the shell (3) is not in contact with, and is in particular at a spacing from, the housing inner wall in a plurality of second regions (10) in the direction of extent of the hollow fibres, and the first and second regions (9, 10) surround the bundle, in particular with a uniform angle distribution, wherein a plurality of the plurality of hollow spaces formed in the second regions (10) between shell (3) and housing (6) each lead through the housing wall into a fluid connection (11) through which the respective hollow space can be provided with a negative pressure relative to the internal pressure in the chamber (1), and the elastic shell, on account of its elastic deformability, can be moved into the hollow space and in this way the chamber volume can be increased.

2. Apparatus according to Claim 1, wherein the bundle with the shell (3) has a substantially circular external cross section, and the surrounding housing (6) is adapted to this cross section, in particular being of identical shape, and has in the inner housing wall, preferably with a uniform angle distribution, a plurality of recesses or indents that form second regions (10).

3. Apparatus according to Claim 1, wherein the bundle with the shell (3) has a substantially circular external cross section, and the housing (6) has a substantially polygonal internal cross section, wherein second regions are formed, in the direction of extent of the hollow fibres, between the corner regions of the housing inner wall and the shell.

4. Apparatus according to one of the preceding claims, wherein, in two opposite second regions (10), connections (4) integrally formed on the shell (3) for supplying and withdrawing the first medium through recesses (7) in the housing (6) are routed through the housing wall, in particular wherein a one-way valve or an active valve, in particular one that compresses and releases the hose from the outside, is arranged in at least one of the connections (4).

5. Apparatus according to Claim 4, wherein, in two apposite second regions (10), preferably in all the remaining second regions (10), the hollow spaces formed between shell (3) and housing (6) lead through the housing wall into a fluid connection (11), in particular a fluid connection (11) that can be subjected to negative pressure and/or positive pressure.

6. Apparatus according to one of the preceding claims, wherein an even number of second regions (10) is formed between the housing inner wall and the shell (3), wherein in particular the internal cross section of the housing perpendicular to the direction of extent of the hollow fibres is polygonal with an even number of corners.

7. Apparatus according to one of the preceding claims, wherein projections (5) extending in the direction of the hollow fibres are arranged on the shell (3) in the first regions (9) and each lie, in particular with form-fit engagement, in a respective groove (8) extending in the housing inner wall in the direction of the hollow fibres.

8. Apparatus according to one of the preceding claims, wherein the bundle of hollow fibres is wound onto a core (13).

9. Apparatus according to Claim 8, wherein the core (13) is hollow, wherein the outer core wall facing towards the bundle has, at least in some parts, openings (14) which lead to the hollow core interior and which are sealed/cavered by a flexible membrane (15), and wherein the core interior has a fluid connection, in particular one via which the core interior can be subjected to negative pressure and/or positive pressure.

10. Apparatus according to Claim 8 or 9, wherein the core (13) has a polygonal external cross section perpendicular to the direction of extent of the hollow fibres, wherein openings (14) are arranged in the flat surface regions between the edges of the core (13) that extend in the direction of the hollow fibres, in particular wherein the core (13) has, in cross section, a number of corners equal to that of the housing.

11. Apparatus according to one of Claims 8 to 10, wherein the core carries a flexible elastic shell (15).

## Revendications

1. Dispositif pour l'échange de matière et/ou d'énergie entre deux fluides, en particulier entre du sang et un gaz/mélange gazeux, avec une chambre (1) pouvant être traversée par le premier fluide, en particulier par du sang, dans laquelle un faisceau de fibres creuses perméables à la matière et/ou à l'énergie est disposé transversalement à la direction de passage du premier fluide, qui peuvent être parcourues par le deuxième fluide et balayées par le premier fluide, dans lequel la chambre (1) est réalisée sous forme d'enveloppe élastique (3), au moins dans une région qui entoure entièrement le faisceau, et un boîtier rigide (6) est disposé autour de l'enveloppe élastique (3), dont la paroi intérieure de boîtier est en contact avec l'enveloppe (3) dans plusieurs premières zones (9), **caractérisé en ce que** lesdites plusieurs premières zones (9) sont étendues respectivement dans la direction des fibres creuses et l'enveloppe (3) n'est pas en contact avec, en particulier est espacée de la paroi intérieure du boîtier dans plusieurs deuxièmes zones (10) dans la direction d`extension des fibres creuses et les premières et les deuxièmes zones (9, 10) entourent le faisceau, en particulier selon une répartition angulaire uniforme, dans lequel plusieurs des plusieurs cavités formées dans les deuxièmes zones (10) entre l'enveloppe (3) et le boîtier (6) débouchent à travers la paroi du boîtier respectivement dans un raccord de fluide (11), à travers lequel la cavité respective peut être soumisse à une dépression par rapport à la pression intérieure dans la chambre (1) et l'enveloppe élastique peut être déplacée dans la cavité en raison de sa déformabilité élastique et le volume de la chambre peut ainsi être augmenté.

2. Dispositif selon la revendication 1, dans lequel le faisceau avec l'enveloppe (3) présente une section transversale extérieure essentiellement circulaire et le boîtier qui l'entoure (6) est adapté à cette section transversale, en particulier sous la même forme, et présente dans la paroi intérieure du boîtier, de préférence avec une répartition angulaire uniforme, plusieurs évidements ou retraits, qui forment des deuxièmes zones (10).

3. Dispositif selon la revendication 1, dans lequel le faisceau avec l'enveloppe (3) présente une section transversale extérieure de forme essentiellement circulaire et le boîtier (6) présente une section transversale intérieure essentiellement polygonale, dans lequel des deuxièmes zones sont formées entre les régions des sommets de la paroi intérieure du boîtier et l'enveloppe dans la direction de l'extension des fibres creuses.

4. Dispositif selon l'une quelconque des revendications précédentes, dans lequel des raccords (4) formés dans des deuxièmes zones opposées (10) sur l'enveloppe (3) pour l'arrivée et le départ du premier fluide sont menés par des évidements (7) dans le boîtier (6) à travers la paroi du boîtier, en particulier dans lequel une soupape à une voie ou une soupape active, en particulier qui comprime et libère le tuyau de l'extérieur, est disposée dans au moins un des raccords (4).

5. Dispositif selon la revendication 4, dans lequel les cavités formées entre l'enveloppe (3) et le boîtier (6) dans deux, de préférence dans toutes les deuxièmes zones opposées restantes (10), débouchent à travers la paroi du boîtier dans un raccord de fluide (11), qui peut en particulier être soumis à une dépression et/ou à une surpression.

6. Dispositif selon l'une quelconque des revendications précédentes, dans lequel un nombre pair de deuxièmes zones (10) sont formées entre la paroi intérieure du boîtier et l'enveloppe (3), en particulier la section transversale intérieure du boîtier perpendiculaire à la direction de l'extension des fibres creuses est polygonale avec un nombre pair de sommets.

7. Dispositif selon l'une quelconque des revendications précédentes, dans lequel des saillies (5) s'étendant dans la direction des fibres creuses sont disposées dans les premières zones (9) sur l'enveloppe (3), et s'engagent notamment par emboîtement dans une rainure (8) s'étendant dans la paroi intérieure du boîtier dans la direction des fibres creuses.

8. Dispositif selon l'une quelconque des revendications précédentes, dans lequel le faisceau des fibres creuses est enroulé sur un noyau (13).

9. Dispositif selon la revendication 8, dans lequel le noyau (13) est creux, dans lequel la paroi extérieure du noyau tournée vers le faisceau présente au moins localement des passages (14) vers l'intérieur du noyau creux, qui sont fermés/recouverts par une membrane flexible (15) et dans lequel l'intérieur du noyau présente un raccord de fluide, en particulier par lequel l'intérieur du noyau peut être soumis à une dépression et/ou à une surpression.

10. Dispositif selon la revendication 8 ou 9, dans lequel le noyau (13) présente perpendiculairement à la direction de l'extension des fibres creuses une section transversale extérieure polygonale, dans lequel des passages (14) sont disposés dans des zones plates planes entre les arêtes du noyau (13) s'étendant dans la direction des fibres creuses, en particulier dans lequel le noyau (13) comporte en section transversale un nombre de sommets égal à celui du boîtier.

11. Dispositif selon l'une quelconque des revendications 8 à 10, dans lequel le noyau porte une enveloppe élastique flexible (15).
